# EUROPEAN PATENT SPECIFICATION

(11) **EP 1 964 503 B1**
(45) Date of publication and mention of the grant of the patent: **20.07.2011**
(21) Application number: 06822818.8
(22) Date of filing: 01.11.2006
(51) Int. Cl.: A61B 1/00

(54) **ENDOSCOPE APPARATUS**
ENDOSKOPIEGERÄT
APPAREIL ENDOSCOPIQUE

(30) Priority: 21.12.2005 JP 2005368684
(43) Date of publication of application: 03.09.2008
(73) Proprietor: OLYMPUS MEDICAL SYSTEMS CORP., Tokyo 151-0072 (JP)
(72) Inventor: TAKEI, Shunji, Tokyo 151-0072 (JP)
(74) Representative: von Hellfeld, Axel
(86) International application number: PCT/JP2006/321889
(87) International publication number: WO 2007/072633

(56) References cited:
- EP-A- 1 294 186
- EP-A- 1 467 197
- JP-A- 2001 029 313
- JP-A- 2003 010 101
- JP-A- 2003 079 570
- US-A1- 2002 022 766

## Description

### Technical Field

The present invention relates to an endoscope apparatus capable of picking up a fluorescence image and a normal image.

### Background Art

Generally, in a frame sequential type electronic endoscope apparatus capable of picking up a fluorescence image and an ordinary image, the intensity of self-fluorescence emitted from a biological mucosa by irradiation of excitation light from a light source device is very weak as compared with the intensity of reflection light from the mucosa obtained by irradiation of non-excitation light. Therefore, when a fluorescence image and a normal image are picked up at the same time, if the light quantities of the excitation light and non-excitation light emitted from the light source are controlled with the same diaphragm mechanism to suppress the light quantity of the non-excitation light, there arises the problem that the light quantity of the excitation light is suppressed at the same time and it becomes difficult to obtain the fluorescence image with sufficient brightness.

In order to cope with this, various proposals have been conventionally made in regard to brightness adjustment of a fluorescence image and a normal image. For example, Japanese Patent Application Laid-Open Publication No. 2002-95635 (Patent Document 1) proposes the art of adjusting light quantities individually by including diaphragm mechanisms for two light source lamps respectively. Further, Japanese Patent Application Laid-Open Publication No. 2003-10101 (Patent Document 2) proposes the art in which in an analog signal which is photoelectrically converted in an image pickup device, the signal of a fluorescence image is amplified with a larger gain than that of the signal of a normal image, and thereafter, the signal of the fluorescence image is subjected to digital conversion.

However, in the art of Patent Document 1, diaphragm mechanisms are included for two light source lamps respectively, and the light quantities are individually adjusted. Therefore, it is necessary to include two light source lamps, and not only the apparatus increases in size, but also the cost is increased.

Further, in the art of Patent Document 2, in the analog signal which is photoelectrically converted in the image pickup device, the signal of the fluorescence image is subjected to digital conversion after being amplified with a larger gain than that of the signal of the normal image. Therefore, the S/N characteristic of the fluorescence image is likely to degrade, and there is the fear that an image with a lot of noise is provided.

EP 1 294 186 discloses an endoscope device which irradiates light of a plurality of specific wavelength regions successively onto a subject by means of a light source device, a solid-state image pickup element capable of changing the charge multiplication rate and which receives and multiplies an optical signal on the basis of the light irradiated on to the subject, and signal processing means which processes the output signal from the solid-state image pickup element, and charge multiplication rate setting means for setting the ratio of charge multiplication rates between the light of a plurality of specific wavelength regions received by the solid-state image pickup element. The endoscope comprises a RGB filter having two sets of filters, one for normal, and one for special light (fluorescence observation), wherein excitation light is filtered from the fluorescence signal in special light observation.

The present invention is made in view of the above described circumstances, and has an object to provide an endoscope apparatus capable of obtaining optimal brightness balance by properly controlling light quantities of both excitation light and non-excitation light emitted from one light source lamp.

### Disclosure of Invention

### Means for Solving the Problem

In order to attain the above described object, a first endoscope apparatus according to claim 1 is provided. The first endoscope apparatus may include, in an endoscope apparatus comprising light emitting means emitting illumination light including an ultraviolet region and a visible region, illumination means including wavelength restricting means restricting a wavelength band of the illumination light to light in the ultraviolet region or the visible region, illumination transmitting means transmitting the illumination light to an observation target, an endoscope insertion section including image pickup means picking up an image of fluorescence or reflection light emitted from the observation target, and image generating means generating a synthetic image of a fluorescence image by the fluorescence arid a normal image by the reflection light based on an image pickup signal obtained by the image pickup means, first light shielding means formed by a material which does not have a transmission property for ultraviolet light and visible light of the wavelength band of the illumination light, on an optical path between the light emitting means and the wavelength restricting means, and second light shielding means having a transmission property for only ultraviolet light, on an optical path between the wavelength restricting means and the first light shielding means.

From the above configuration, the present invention properly controls the light quantities of both the excitation light and non-excitation light emitted from one light source lamp, and optimal brightness balance can be obtained. Especially in the first endoscope apparatus, the light quantities of the excitation light and visible light are adjusted by the first light shielding means, and the light quantity adjustment can be performed only for visible light by the second light shielding means. Therefore, the light quantity of the excitation light and the light quantity of the visible light emitted from one light source lamp are individually controlled, and optimal brightness balance can be obtained. Further, in the second endoscope apparatus, the light quantity of non-excitation light is restricted to the light quantity smaller than the light quantity of the excitation light with the wavelength restricting means, whereby the light quantity of self-fluorescence emitted from the observation target by irradiation of the excitation light and the light quantity of the reflection light by irradiation of non-excitation light can be made equivalent, and optimal brightness balance can be obtained while noise by gain adjustment is suppressed.

### Brief Description of the Drawings

Fig. 1 is a general configuration diagram of an endoscope apparatus according to a first embodiment of the present invention;
Fig. 2 is an explanatory view showing a configuration of an image pickup unit according to the same;
Fig. 3 is an explanatory view showing a configuration of a rotating filter according to the same;
Fig. 4 is a block diagram showing a configuration of an image processing unit according to the same;
Fig. 5 is a general configuration diagram of an endoscope apparatus according to a second embodiment of the present invention;
Fig. 6 is an explanatory view showing a configuration of a rotating filter according to the same;
Fig. 7 is an explanatory diagram showing spectral characteristics of a first switching filter according to the same;
Fig. 8 is an explanatory diagram showing spectral characteristics of a second switching filter according to the same;
Fig. 9 is an explanatory view showing filter disposition at a time of a normal image mode according to the same;
Fig. 10 is an explanatory view showing filter disposition at a time of a simultaneous observation mode according to the same;
Fig. 11 is an explanatory diagram showing irradiation timing of frame sequential light in each observation mode according to the same;
Fig. 12 is an explanatory view showing a configuration of a rotating filter according to a modified example; and
Fig. 13 is an explanatory view showing a configuration of an image pickup unit according to the same.

### Best Mode for Carrying Out the Invention

Hereinafter, embodiments of the present invention will be described with reference to the drawings.

### (First Embodiment)

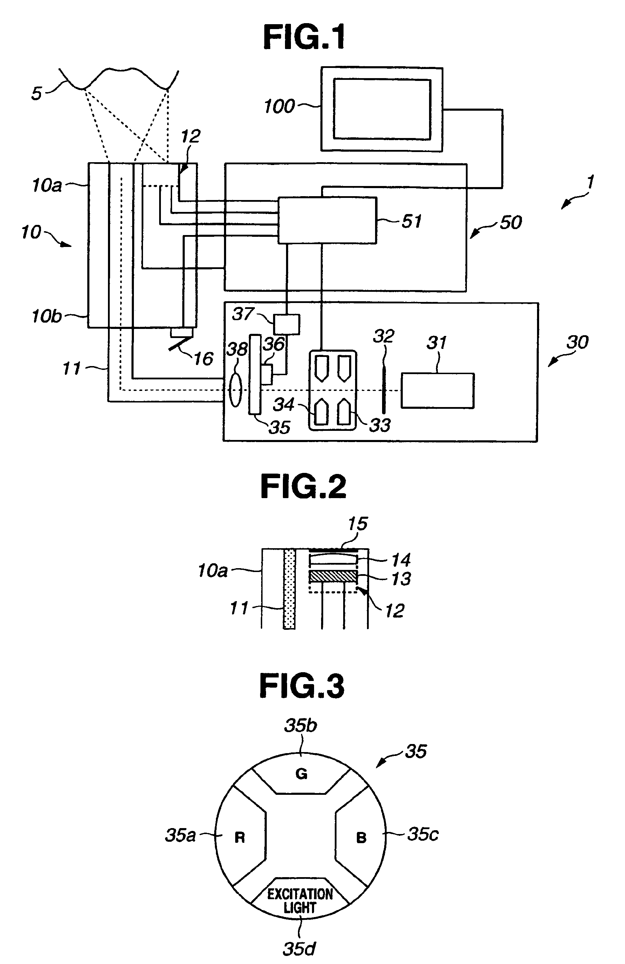

As shown in Fig. 1, an endoscope apparatus 1 of a first embodiment includes an endoscope 10 capable of observation by fluorescence or reflection light emitted from an observation target 5 such as a biological mucosa, a light source device 30 which supplies illumination light for illuminating the observation target 5 to the endoscope 10, a processor 50 which processes an image pickup signal from the endoscope 10 to generate an observation image, and a monitor 100 for displaying the observation image generated by the processor 50.

The endoscope 10 includes an endoscope insertion section 10a which is inserted into a body cavity or the like, and an operation section 10b which is provided at a proximal end side of the endoscope insertion section 10a. An emission end of a light guide 11 as illumination transmitting means for transmitting the illumination light emitted from the light source device 30 to irradiate the observation target 5 with the illumination light, and an image pickup unit 12 as image pickup means for picking up images of fluorescence and reflection light emitted from the observation target 5 by irradiation of the illumination light are disposed at a distal end portion of the endoscope insertion section 10a.

The image pickup unit 12 is configured by disposing an objective lens 14 which condenses incident light on an image pickup device 13 at a front of the image pickup device 13 which photoelectrically converts the incident light of fluorescence and reflection light, and by disposing an excitation light cut filter 15 which cuts a wavelength band of the excitation light of the reflection light when the observation target is irradiated with illumination light at a front of the objective lens 14, as shown in Fig. 2. As the image pickup device 13 which picks up images of fluorescence and reflection light, image pickup devices such as a CCD (Charge Coupled Device) and a CMD (Charge Multiplying Detector), for example, are used.

Further, a mode changeover switch 16 which performs a switching operation between a normal image mode for displaying only a normal image by irradiation of red, green and blue light, and a simultaneous observation mode for simultaneously displaying a fluorescence image by irradiation of excitation light and a normal image by irradiation of light of red, green and blue is placed at the operation section 10b of the endoscope 10.

Hereinafter, light of red, green and blue will be described as RGB light, and reflection light when the observation target is irradiated with light of red, green and blue will be described as RGB reflection light.

Next, in the present embodiment, the light source device 30 includes a light source lamp 31 as light emitting means which is formed as illumination means for irradiating the observation target with illumination light including excitation light of a wavelength band in an ultraviolet region and light in a visible region, and is capable of emitting illumination light having spectra in the ultraviolet region and the visible region, a heat ray cut filter 32 which cuts heat rays of the illumination light, a first diaphragm mechanism 33 and a second diaphragm mechanism 34 disposed on an optical path of the illumination light which adjust the light quantity of the illumination light, a rotating filter 35 as wavelength restricting means which restricts the illumination light to RGB light or excitation light having a wavelength band in an ultraviolet region by being disposed on an optical path of the illumination light and performing a rotational operation, a motor 36 which rotationally operates the rotating filter 35, a drive control circuit 37 which controls drive of the motor 36, and a condenser lens 38 which condenses excitation light and RGB light.

The first diaphragm mechanism 33 is configured by a filter material having absorption characteristics or reflection characteristics for light in the ultraviolet region and the visible region, and forms first light shielding means configured by a material which does not have a transmission property for ultraviolet light and visible light. Further, the second diaphragm mechanism 34 is configured by a filter material which transmits the light in the ultraviolet region, and has absorption characteristics or reflection characteristics for only the light in the visible region, and forms second light shielding means having a transmission property for only ultraviolet light.

As shown in Fig. 3, the rotating filter 35 includes band-pass filters 35a, 35b and 35c which transmit RGB light, and a band-pass filter 35d which transmits the excitation light in the ultraviolet region, and is formed by concentrically disposing the band-pass filters 35a to 35d. The rotating filter 35 is rotationally driven by the motor 36, as a result of which, the kind of filter which is disposed on the optical path of the illumination light is switched in sequence, and the light which passes through several kinds of filters differing in spectral characteristics is intermittently irradiated. The light which is intermittently irradiated through the rotating filter 35 will be referred to as frame sequential light hereinafter.

Meanwhile, the processor 50 mainly includes an image processing unit 51 as image creating means which processes an image pickup signal from the image pickup unit 12, and creates a synthetic image of a normal image by RGB reflection light from an observation target and a fluorescence image by fluorescence emitted from the observation target.

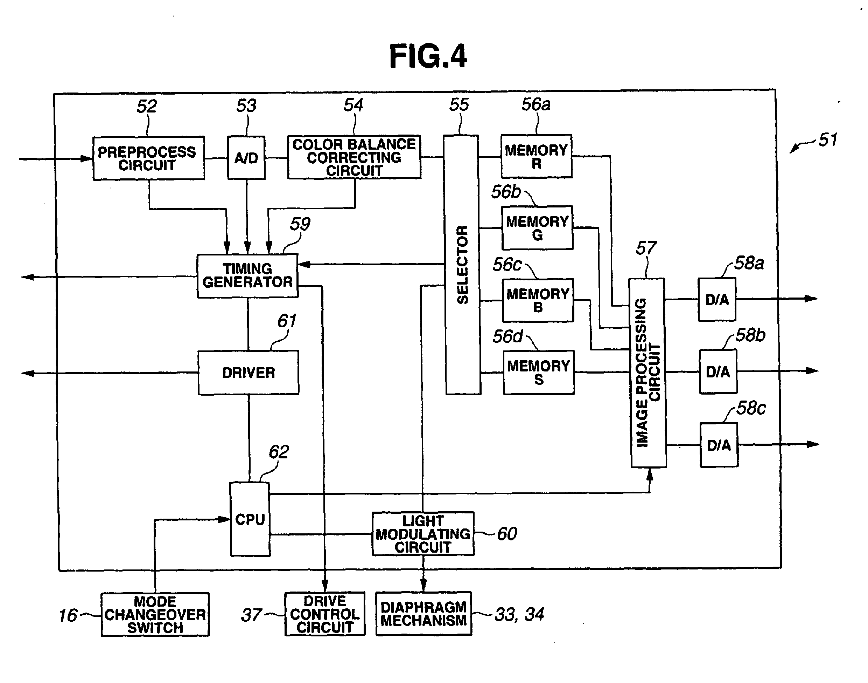

As the details are shown in Fig. 4, the image processing unit 51 includes a preprocess circuit 52 which performs noise removal of an image pickup signal and the like, an A/D converter 53 which converts an analog image pickup signal into a digital signal, a color balance correcting circuit 54 for performing color balance correction based on a digital signal, a selector 55 for separating digital signals in accordance with the kinds of illumination light, four memories 56a, 56b, 56c and 56d for synchronizing the separated signals, an image processing circuit 57 for generating two kinds of observation images and character information by performing arithmetic processing for the signals synchronized in the four memories 56a to 56d, and D/A converters 58a, 58b and 58c for converting the arithmetically processed digital signals into analog video signals.

Further, the image processing unit 51 includes a timing generator 59 which synchronizes timing of image pickup by the image pickup device 13 of the image pickup unit 12 and illuminating timing of illumination light by the rotating filter 35, a light modulating circuit 60 which controls the operation of the diaphragm mechanism (the first diaphragm mechanism 33 and the second diaphragm mechanism 34) of the light source device 30 based on the image pickup signal, a driver 61 which drives the image pickup device 13, and a CPU 62 which detects the operation of the mode changeover switch 16 and performs a switching operation between a normal image mode and a simultaneous observation mode.

Next, an operation of the endoscope 1 having the above configuration will be described. When illumination light having spectra in the ultraviolet region and the visible light region is emitted from the light source lamp 31 contained in the light source device 30 first, the illumination light is incident on the heat ray cut filter 32. The heat ray cut filter 32 shields the heat rays included in the illumination light, and thereby, heat generation of the observation target is suppressed.

The illumination light from which the heat rays are cut with the heat ray cut filter 32 has its light quantity adjusted by the first diaphragm mechanism 33 and the second diaphragm mechanism 34. The first diaphragm mechanism 33 and the second diaphragm mechanism 34 are controlled based on a signal from the light modulating circuit 60 inside the processor 50, and adjusts the light quantity of the illumination light by being narrowed by being inserted into a pass region of light. In detail, the first diaphragm mechanism 33 is a mechanism which adjusts the light quantities of excitation light and RGB light of the wavelength band of the illumination light by performing an opening and a closing operations on an optical path of the illumination light, and the second diaphragm mechanism 34 is a mechanism which adjusts the light quantity of RGB light without influencing the light quantity of the excitation light since the second diaphragm mechanism 34 has a transmission property for the light in the ultraviolet region.

Further, the illumination light of which light quantity is adjusted by the first diaphragm mechanism 33 and the second diaphragm mechanism 34 is incident on the rotating filter 35 disposed on the optical path, and is emitted as frame sequential light from the rotating filter 35. Specifically, the rotating filter 35 rotationally drives by the operation of the motor 36 which receives a control signal from the drive control circuit 37, and the band-pass filters 35a to 35d differing in spectral characteristics are sequentially disposed and switched on the optical path of the illumination light, whereby the RGB light and excitation light are sequentially incident on the incident end of the light guide 11 via the condenser lens 38.

The frame sequential light from the light source device 30 is guided by the light guide 11 and irradiated to the observation target from the emission end of the distal end of the endoscope insertion section 10a, and an image of self-fluorescence or reflection light which is obtained from the observation target by irradiation of the frame sequential light is picked up by the image pickup unit 12 contained in the distal end of the endoscope insertion section 10a. Image pickup by the image pickup unit 12 is controlled by the timing generator 59 inside the processor 50 and is performed in synchronism with the illumination timing of the frame sequential light.

In the time zone in which the excitation light is irradiated, of the self-fluorescence which is emitted from the observation target and reflection light of the excitation light, reflection of the excitation light having spectra in a near ultraviolet region is shield by the excitation light cut filter 15, and only the self-fluorescence having spectra in the visible light region is condensed by the objective lens 14 and photoelectrically converted by the image pickup device 13.

Further, at the time of irradiation of RGB light, RGB reflection light having spectra in the visible light region is photoelectrically converted by the image pickup device 13 without being shielded by the excitation light cut filter 15. The image pickup signal generated in the image pickup device 13 by the photoelectric conversion is subjected to level adjustment by amplifying processing in an analog form in the image processing unit 51 inside the processor 50.

As shown in Fig. 4, the image processing unit 51 performs noise removal and gain adjustment for the image signals sent from the image pickup device 13 in the preprocess circuit 52, converts the signals into digital signals via the A/D converter 53, and performs gain adjustment for the signals in the color balance correcting circuit 54. Thereafter, the image processing unit 51 separates the signals in the selector 55, and stores them in the four memories 56a to 56d for RGB light and fluorescence (R, G, B, S). At this time, the image pickup signals which are continuously sent are separated based on the irradiation timing of the frame sequential light in the selector 55, and after the separated image pickup signals are synchronized in the memories 56a to 56d, the signals are outputted to the image processing circuit 57.

The image processing circuit 57 generates a normal image by the RGB light and a fluorescence image by the excitation light based on the image pickup signal sent from each of the memories 56a to 56d, and separates or synthesizes parts of them, and after adding character information to it, the image processing circuit 57 outputs it to a monitor 100 as the image data. At this time, the monitor 100 receives the image data from the image processing unit 51, and displays an observation image for an operator of the endoscope apparatus 1 in the present embodiment to observe the observation target as a color image.

At this time, the timing generator 59 receives the synchronous signal which is sent from the drive control circuit 37 of the rotating filter 35, and controls the driver 61 for driving the image pickup device 13 so that the image pickup device 13 picks up an image in synchronism with the irradiation timing of the illumination light.

Further, in the image processing unit 51, the CPU 62 detects the switching operation between the normal image mode and the simultaneous observation mode by the mode changeover switch 16 of the endoscope 10, and transmits a switching signal to the image processing circuit 57. When the image processing circuit 57 receives the switching signal indicating that the normal image mode is selected from the CPU 62, the image processing circuit 57 generates a normal image based on the three image pickup signals by RGB light stored in the memories 56a to 56c. Further, when the image processing circuit 57 receives the switching signal indicating that the simultaneous observation mode is selected from the CPU 62, the image processing circuit 57 receives four image pickup signals (four synchronized image pickup signals) by RGB reflection light and fluorescence stored in the memories 56a to 56d of R, G, B and S, and generates a synthetic image of the normal image and the fluorescence image.

The switching signal from the CPU 62 which detects the mode switching operation is sent to the driver 61 and the light modulating circuit 60 at the same time. The driver 61 controls the drive of the image pickup device 13 based on the switching signal from the CPU 62. Further, the light modulating circuit 60 controls the first diaphragm mechanism 33 and the second diaphragm mechanism 34 based on the switching signal from the CPU 62 and a light modulating signal from the preprocess circuit 52 via the selector 55.

Next, the process in which the preprocess circuit 52 and the light modulating circuit 60, and the first diaphragm mechanism 33 and the second diaphragm mechanism 34 perform light quantity adjustment of the illumination light will be described.

The preprocess circuit 52 generates an information signal relating to the brightness of an image which the image pickup signal has. The light modulating circuit 60 generates a light modulating signal for controlling the first diaphragm mechanism 33 and the second diaphragm mechanism 34 based on the information signal relating to the brightness of the image. The information signal relating to the brightness of the image and the light modulating signal based on the information signal are generated in the respective image pickup signals of RGB reflection light and fluorescence.

The first diaphragm mechanism 33 performs light quantity adjustment of the irradiation light of the excitation light based on the light modulating signal of fluorescence. At this time, the first diaphragm mechanism 33 has the diaphragm effect for the light quantity of RGB light and for excitation light at the same time, but self-fluorescence emitted from an observation target generally has a very small light quantity as compared with RGB reflection light, and in order to adjust the RGB light to an optimal light quantity, only the light quantity restriction by the first diaphragm mechanism 33 is insufficient.

In contrast to the above, the second diaphragm mechanism 34 receives the light modulating signal based on the image pickup signal of RGB reflection light, and adjusts the RGB illumination light emitted from the light source device 30 to have an optimal light quantity. At this time, the second diaphragm mechanism 34 has a transmission property for the excitation light in the ultraviolet region, and therefore, does not have an influence on the light quantity of the excitation light adjusted by the first diaphragm mechanism 33.

As above, in the endoscope apparatus 1 of the first embodiment, by sequentially irradiating the excitation light and RGB light by passing the illumination light emitted from the light source lamp 31 through the rotating filter 35, the fluorescence image and normal image obtained from the observation target are simultaneously picked up, and can be displayed on the monitor 100. The observation target in this case is mainly a biological tissue of a bronchus, digestive organ or the like. Intensity of the self-fluorescence emitted from a biological tissue is generally very weak as compared with RGB reflection light, and in order to keep brightness of the image constant, the light quantity adjustment needs to be performed individually for the excitation light and RGB light. In this regard, the first diaphragm mechanism 33 in the present embodiment has the function of adjusting the light quantities of the excitation light in the ultraviolet region and RGB light in the visible light region, and the second diaphragm mechanism 34 performs light quantity adjustment for only the RGB light in the visible light region. Therefore, the light quantities of both the excitation light and RGB light emitted from one light source lamp are properly controlled, and optimal brightness balance can be obtained.

Further, when excitation light and RGB light are controlled with the same diaphragm mechanism in accordance with the brightness of a fluorescence image or a normal image, the light quantity of either the excitation light or RGB light is restricted more than necessary, and therefore, gain adjustment needs to be performed for image pickup signals. Thus, the S/N characteristic of one of the images degrades, and the image with a lot of noise is provided. On the other hand, the endoscope apparatus 1 in the present embodiment can individually modulate excitation light and illumination light. Therefore, the endoscope apparatus 1 can ensure the optimal light quantity for each of the fluorescence image and normal image, and reduces gain adjustment for the image pickup signals to be able to obtain images with less noise.

For example, when an image is picked up at the position in which the distal end of the endoscope insertion section 10a is close to the observation target 5 in the endoscope apparatus 1 of Fig. 1, sufficient light quantities of fluorescence and RGB reflection light for observation are obtained, and they are adjusted to proper light quantities respectively by the first diaphragm mechanism 33 and the second diaphragm mechanism 34. Therefore, a fluorescence image and a normal image with less noise can be obtained without performing gain adjustment for image pickup signals inside the processor 50.

Meanwhile, when the distance from the distal end of the endoscope insertion section 10a to the observation target 5 is long, and a sufficient quantity of fluorescence for observation cannot be obtained even if the maximum light quantity of excitation light is irradiated, gain adjustment needs to be performed for the image pickup signal by the fluorescence. However, in this case, RGB reflection light which is obtained in a sufficient light quantity is adjusted to a proper light quantity by the second diaphragm mechanism 34. Therefore, gain adjustment does not need to be performed, and a normal image with less noise can be obtained.

Further, the diaphragm mechanisms do not need to be provided individually for the excitation light and RGB light. Therefore, a mechanism which separates the illumination light irradiated from one light source lamp into excitation light in the ultraviolet region and RGB light in the visible region does not need to be provided, or two exclusive light source lamps do not need to be provided for the excitation light and RGB light, and therefore, the effect of suppressing increase in size of the light source device and increase in cost can be obtained.

### (Second embodiment)

Next, a second embodiment of the present invention will be described. In contrast with the aforementioned first embodiment, the second embodiment uses a diaphragm mechanism 65 which shields part of excitation light and RGB light, instead of the first diaphragm mechanism 33 and the second diaphragm mechanism 34, and in order to avoid the aforementioned problem of the case of controlling excitation light and RGB light with the same diaphragm mechanism, that is, the problem that gain adjustment for an image pickup signal is performed to cope with the situation that the light quantity of either the excitation light or RGB light is restricted more than necessary, as a result of which, the S/N characteristic of one of the images degrades, and an image with a lot of noise is provided, the configuration of the rotating filter 35 is changed.

The same members and circuit sections as those in the first embodiment will be assigned with the same reference numerals, and the explanation of them will be omitted.

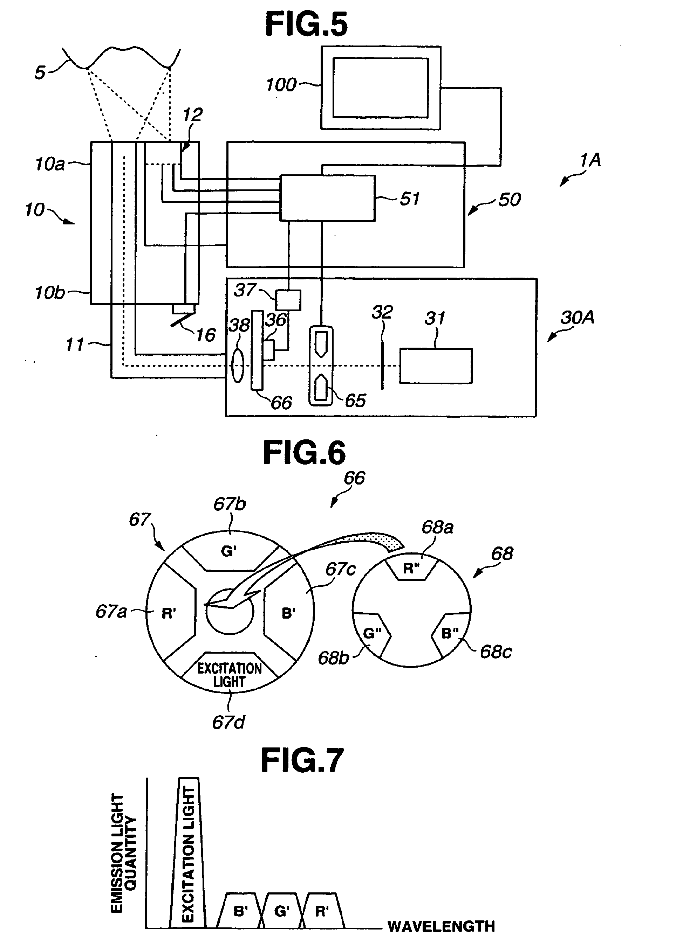

As shown in Fig. 5, an endoscope apparatus 1A of the second embodiment has substantially the same configuration as the endoscope apparatus 1 in the first embodiment, and is configured by including the endoscope 10, and the diaphragm mechanism 65 which shields part of excitation light and RGB light instead of the first diaphragm mechanism 33 and the second diaphragm mechanism 34 in the first embodiment, and also including a light source device 30A including a rotating filter 66 including different filter groups in its outer circumference and inner circumference instead of the rotating filter 35, the processor 50 and the monitor 100.

The diaphragm mechanism 65 of the light source device 30A is configured by a material which does not have a transmission property for both excitation light and RGB light. Further, the rotating filter 66 is configured by a first switching filter 67 at an outer circumferential side, and a second switching filter 68 which is formed to have a smaller diameter than the first switching filter 67 to be concentrically disposed at an inner circumferential side, as shown in Fig. 6.

The first switching filter 67 has the configuration in which four band-pass filters 67a, 67b, 67c and 67d which transmit RGB light and excitation light are concentrically disposed, and the second switching filter 68 at the inner circumferential side has the configuration in which three band-pass filters 68a, 68b and 68c which transmit RGB light are concentrically disposed.

Among the four band-pass filters 67a to 67d of the first switching filter 67, the band-pass filters 67a, 67b and 67c which transmit RGB light have transmittances lower than that of the band-pass filter 67d which transmits excitation light, so that the light quantities of the RGB light (R', G', B') which pass through the band-pass filters 67a, 67b and 67c are set to be small with respect to the light quantity of the excitation light which passes through the band-pass filter 67d, as shown in Fig. 7.

The wavelength band of the excitation light in the second embodiment includes the wavelength band in not only the ultraviolet region but also in a blue visible light region, unlike the first embodiment.

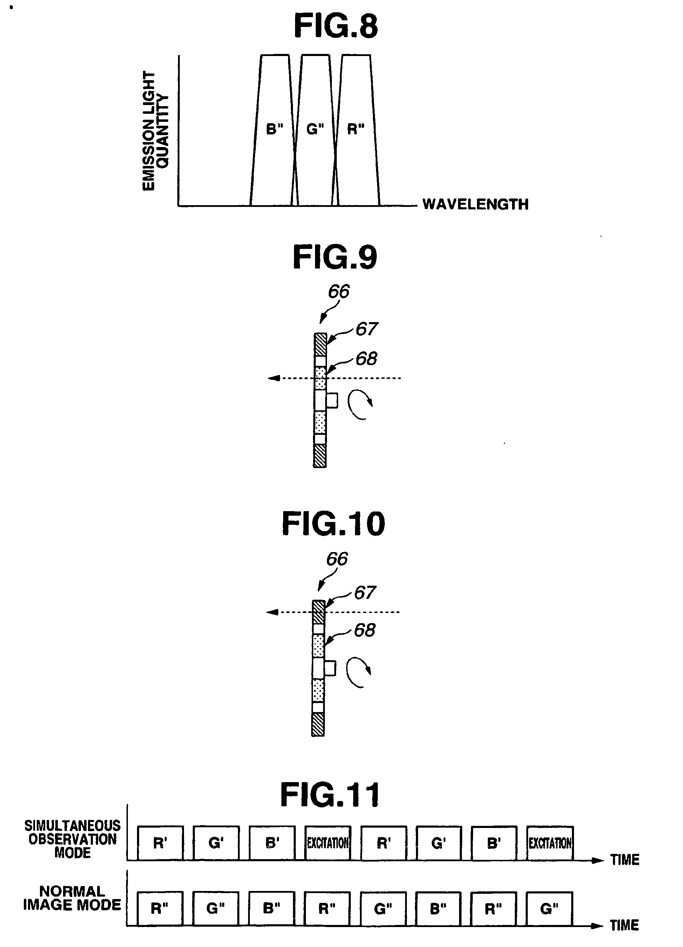

Further, the three band-pass filters 68a to 68c of the second switching filter 68 are set to have the characteristics that transmit larger quantities of RGB light (R", G", B") than the RGB filters (band-pass filters 67a to 67c) of the first switching filter 67 as shown in Fig. 8.

An operation of the endoscope apparatus 1 A in the second embodiment having the above configuration is substantially the same as that of the first embodiment, and only a different point about the operation relating to the rotating filter 66 and the diaphragm mechanism 65 in the normal image mode and the simultaneous observation mode will be described.

When the CPU 62 included in the image processing unit 51 of the processor 50 detects the switching operation between the normal image mode and the simultaneous observation mode by the mode changeover switch 16, and generates a switching signal, the switching signal is transmitted to the drive control circuit 37 of the motor 36 contained in the light source device 30A via the timing generator 59. The drive control circuit 37 switches the disposition of the rotating filter 66 with respect to the optical path of the illumination light in accordance with the selected mode. The operation at the time of receiving the switching signal in the image processing circuit 57 is the same as in the first embodiment.

For example, when the drive control circuit 37 receives the switching signal indicating that the normal image mode is selected, the drive control circuit 37 controls the drive of the motor 36 so that the filter group of the second switching filter 68 in the inner circumference of the rotating filter 66 moves onto the optical path of the illumination light, as shown in Fig. 9. At this time, the illumination light which passes through the filter group of the second switching filter 68 is transmitted to the observation target by the light guide 11 as the light transmitting means and irradiated as the frame sequential light of R"G"B" with large light quantities as shown in Fig. 11.

When the switching signal indicating that the simultaneous observation mode is selected is received in the drive control circuit 37, the drive control circuit 37 controls the drive of the motor 36 so that the filter group of the first switching filter 67 at the outer circumference of the rotating filter 66 is moved onto the optical path of the illumination light as shown in Fig. 10. At this time, as the illumination light, the excitation light and the frame sequential light of R'G'B' with smaller light quantities than the excitation light is irradiated to the observation target as shown in Fig. 11.

Specifically, the rotating filter 66 as the wavelength restricting means in the second embodiment realizes the function of restricting the wavelength band of the illumination light to the excitation light for obtaining a fluorescence image and non-excitation light for obtaining a normal image by the first switching filter 67 at the outer circumferential side, and restricting the light quantity of the non-excitation light to the light quantity which is smaller than the light quantity of the excitation light.

Meanwhile, the diaphragm mechanism 65 in the second embodiment adjusts the light quantity by shielding part of the illumination light based on the light modulating signal from the light modulating circuit 60 which is contained in the image processing unit 51. As described above, the diaphragm mechanism 65 in the second embodiment is configured by the material which does not have a transmission property for both the excitation light and RGB light, and forms light quantity adjusting means which adjusts the light quantity of the illumination light.

As above, in the endoscope apparatus 1A of the second embodiment, the rotating filter 66 is configured by arranging the RGB filter with a lower transmittance than the filter for excitation light in the filter group at the outer circumference so that the light quantities of fluorescence emitted from the observation target and RGB reflection light become equivalent light quantities in the simultaneous observation mode, and the fluorescence and RGB reflection light which are incident on the image pickup device 13 have substantially equal light quantities. Accordingly, it is not necessary to perform light quantity adjustment individually for the excitation light for exciting fluorescence and RGB light, the light quantities of both the excitation light and RGB light emitted from one light source lamp are controlled by the one diaphragm mechanism 65, and optimal brightness balance can be obtained as in the first embodiment.

In this case, when the light quantity of fluorescence incident on the image pickup device is very small with respect to the light quantity of RGB reflection light, in the configuration of controlling the excitation light and RGB light with the same diaphragm mechanism, the light quantity is suppressed to avoid saturation of the RGB reflection light in the image pickup device, and at the same time the light quantity of fluorescence becomes extremely small. Therefore, gain adjustment needs to be performed inside the processor. Thus, there arises the problem that the S/N characteristic of the fluorescence image degrades and an image with a lot of noise is provided. On the other hand, in the second embodiment, the fluorescence and RGB reflection light incident on the image pickup device have substantially equal light quantities. Therefore, even if the light quantity of the RGB reflection light is suppressed, the light quantity of the fluorescence does not become extremely small, and there is provided the advantage that an increase in noise due to gain adjustment is small.

The present invention is not limited to the above described respective embodiments, and various modifications, alterations and the like are possible within the range without changing the gist of the present invention.

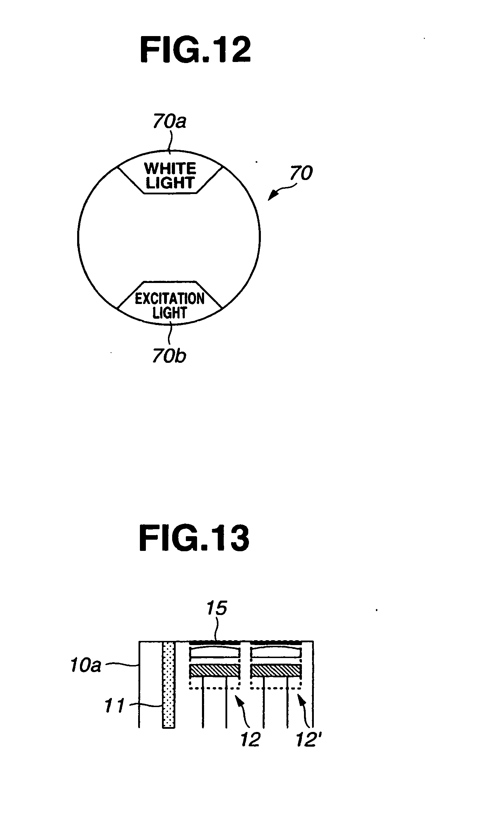

For example, in the image pickup unit 12 in each of the above embodiments, a color filter in a mosaic form may be included instead of the excitation light cut filter 15 disposed at the front of the image pickup device 13 (front of the objective lens 14). In the case of adopting the mosaic color filter, a rotating filter 70 in which two band-pass filters 70b and 70a which transmit wavelength bands of the excitation light for obtaining a fluorescence image and white light for obtaining a normal image are concentrically arranged as shown in Fig. 12 is included inside the light source device 30 (30A).

By the rotational operation of the rotating filter 70, excitation light and white light are irradiated as frame sequential light, and the image pickup device 13 picks up images in synchronism with irradiation timings of the excitation light and white light by the timing generator 59. The light which passes through the mosaic color filter is received in each pixel of the image pickup device 13, and the image pickup signal in each pixel is arithmetically processed in the image processing circuit 57, whereby a color image is generated.

Further, in each of the embodiments, the example in which one image pickup unit 12 is included at the distal end portion of the endoscope insertion section 10a is described, but as shown in Fig. 13, two image pickup units that are the image pickup unit 12 and an image pickup unit 12' may be included. The image pickup unit 12 having the excitation light cut filter 15 at the front is used for picking up images of fluorescence, and the image pickup unit 12' which does not have an excitation light cut filter is used for picking up images of RGB reflection light.

## Claims

1. An endoscope apparatus (1) comprising:
light emitting means (31) emitting illumination light including an ultraviolet region and a visible region,
illumination means (30) including wavelength restricting means (35) restricting a wavelength band of the illumination light to light in the ultraviolet region or the visible region,
illumination transmitting means (11) transmitting the illumination light to an observation target (5),
an endoscope insertion section (10a) including image pickup means (12) picking up an image of fluorescence or reflection light emitted from the observation target (5), and
image generating means (51) generating a synthetic image of a fluorescence image by the fluorescence and a normal image by the reflection light based on an image pickup signal obtained by the image pickup means (12), comprising:
first light shielding means (33), which is formed by a material that does not have a transmission property for ultraviolet light and visible light of the wavelength band of the illumination light, on an optical path between the light emitting means (31) and the wavelength restricting means (35);
**characterized by** further comprising second light shielding means (34), which has a transmission property for only the ultraviolet light, on an optical path between the wavelength restricting means (35) and the first light shielding means (33).

2. The endoscope apparatus according to claim 1,
wherein the wavelength restricting means (35) is a rotating filter (35) in which three filters respectively transmitting RGB light, and a filter transmitting ultraviolet light are concentrically disposed.

3. The endoscope apparatus according to claim 2,
wherein the first light shielding means (33) is formed by a first diaphragm mechanism (33) controlled by a light modulating signal generated from the image pickup signal, and adjusts light quantities of the ultraviolet light and RGB light of the wavelength band of the illumination light emitted in frame sequence from the rotating filter (35).

4. The endoscope apparatus according to one of claims 2 and 3,
wherein the second light shielding means (34) is formed by a second diaphragm mechanism controlled by a light modulating signal generated from the image pickup signal, and adjusts a light quantity of RGB light of the illumination light emitted in frame sequence from the rotating filter (35).

5. The endoscope apparatus according to one of claims 3 and 4,
wherein the light modulating signal is generated based on an information signal relating to brightness of an image which the image pickup signal has.

6. The endoscope apparatus according to claim 1,
wherein the image pickup means (12) has an image pickup device (13) photoelectrically converting the fluorescence and the reflection light from the observation target (5), and
a filter (35) cutting a wavelength band of excitation light having spectra in a near ultraviolet region of the reflection light when the observation target (5) is irradiated with the illumination light.

7. The endoscope apparatus according to claim 1, wherein the image pickup means (12) has an image pickup device (13) for picking up images of fluorescence having a filter (35) cutting a wavelength band of excitation light having spectra in a near ultraviolet region of the reflection light when the observation target (5) is irradiated with the illumination light, and for picking up images of RGB reflection light from the observation target (5).

8. The endoscope apparatus according to claim 1, wherein
the wavelength restricting means (35) has a filter (35) transmitting the ultraviolet light and a filter transmitting white light, and
the image pickup means (12) has a color filter in a mosaic form.

9. The endoscope apparatus according to claim 1, further comprising:
a mode changeover switch (16) switching a normal image mode in which the normal image is generated in the image generating means (51) and displayed, and a simultaneous observation mode in which the fluorescence image and the normal image are synthesized in the image generating means (51) and displayed.

## Patentansprüche

1. Endoskopvorrichtung (1), mit:
einem Licht emittierenden Mittel (31), das Beleuchtungslicht mit einem ultravioletten Bereich und einem sichtbaren Bereich emittiert,
einem Beleuchtungsmittel (30), das ein Wellenlängen-Beschränkungsmittel (35) enthält, welches ein Wellenlängenband des Beleuchtungslichts auf Licht in dem ultravioletten Bereich oder dem sichtbaren Bereich beschränkt,
einem Beleuchtungs-Durchlassmittel (11), das das Beleuchtungslicht an ein Beobachtungsziel (5) durchlässt,
einem Endoskop-Einführabschnitt (10a) mit einem Bildaufnahmemittel (12), das ein Bild des Fluoreszenz- oder Reflexionslichtes aufnimmt, das vom Beobachtungsziel (5) emittiert wird, und
einem Bilderzeugungsmittel (51), das ein synthetisches Bild eines Fluoreszenzbildes durch die Fluoreszenz und ein normales Bild durch das Reflexionslicht auf Basis eines Bildaufnahmesignals erzeugt, das vom Bildaufnahmemittel (12) erhalten wird, mit:
einem ersten Lichtabschirmmittel (33), das aus einem Material ohne Durchlasseigenschaft für ultraviolettes und sichtbares Licht des Wellenlängenbandes des Beleuchtungslichtes gebildet ist, in einem optischen Weg zwischen dem Licht emittierenden Mittel (31) und dem Wellenlängen-Beschränkungsmittel (35);
**dadurch gekennzeichnet, dass** es ferner ein zweites Lichtabschirmmittel (34), das eine Durchlasseigenschaft nur für das ultraviolette Licht hat, in einem optischen Weg zwischen dem Wellenlängen-Beschränkungsmittel (35) und dem ersten Lichtabschirmmittel (33) aufweist.

2. Endoskopvorrichtung nach Anspruch 1,
bei der das Wellenlängen-Beschränkungsmittel (35) ein rotierendes Filter (35) ist, in dem drei Filter, die jeweils rotes, grünes und blaues (RGB) Licht durchlassen und ein Filter, das ultraviolettes Licht durchlässt, konzentrisch angeordnet sind.

3. Endoskopvorrichtung nach Anspruch 2,
bei der das erste Lichtabschirmmittel (33) aus einem ersten Membranmechanismus (33) gebildet ist, der von einem Lichtmodulationssignal, das vom Bildaufnahmesignal erzeugt wird, gesteuert wird und Lichtmengen des ultravioletten und des RGB-Lichtes des Wellenlängenbandes des vom rotierenden Filter (35) in Bildsequenz emittierten Beleuchtungslichtes einstellt.

4. Endoskopvorrichtung nach einem der Ansprüche 2 und 3,
bei der das zweite Lichtabschirmmittel (34) aus einem zweiten Membranmechanismus gebildet ist, der von einem Lichtmodulationssignal, das vom Bildaufnahmesignal erzeugt wird, gesteuert wird und eine Lichtmenge des RGB-Lichtes des vom rotierenden Filter (35) in Bildsequenz emittierten Beleuchtungslichtes einstellt.

5. Endoskopvorrichtung nach einem der Ansprüche 3 und 4,
bei der das Lichtmodulationssignal auf Basis eines Informationssignals erzeugt wird, das die Helligkeit eines Bildes betrifft, welche das Bildaufnahmesignal aufweist.

6. Endoskopvorrichtung nach Anspruch 1,
bei der das Bildaufnahmemittel (12) ein Bildaufnahmegerät (13) hat, das die Fluoreszenz und das Reflexionslicht vom Beobachtungsziel (5) fotoelektrisch wandelt, und
und ein Filter (35), das ein Wellenlängenband des Anregungslichtes mit Spektren in einem nahen Ultraviolettbereich des Reflexionslichtes abschneidet, wenn das Beobachtungsziel (5) mit dem Beleuchtungslicht bestrahlt wird.

7. Endoskopvorrichtung nach Anspruch 1,
bei der das Bildaufnahmemittel (12) ein Bildaufnahmegerät (13) zum Aufnehmen von Bildern der Fluoreszenz mit einem Filter (35) hat, das ein Wellenlängenband des Anregungslichtes mit Spektren in einem nahen Ultraviolettbereich des Reflexionslichtes abschneidet, wenn das Beobachtungsziel (5) mit dem Beleuchtungslicht bestrahlt wird, und zum Aufnehmen von Bildern des RGB-Reflexionslichtes vom Beobachtungsziel (5).

8. Endoskopvorrichtung nach Anspruch 1,
bei der das Wellenlängen-Beschränkungsmittel (35) ein Filter (35) hat, das das ultraviolette Licht durchlässt, und ein Filter, das weißes Licht durchlässt, und das Bildaufnahmemittel (12) ein Farbfilter in Mosaikform hat.

9. Endoskopvorrichtung nach Anspruch 1, ferner mit:
einem Modus-Wechselschalter (16), der in einen Normalbildmodus schaltet, in dem das Normalbild im Bilderzeugungsmittel (51) erzeugt wird und angezeigt wird, und in einen simultanen Beobachtungsmodus, in dem das Fluoreszenzbild und das Normalbild im Bilderzeugungsmittel (51) synthetisiert werden und angezeigt werden.

## Revendications

1. Appareil endoscopique (1) comprenant:
un moyen d'émission de lumière (31) émettant une lumière d'éclairage comprenant une région ultraviolet et une région visible,
un moyen d'éclairage (30) comprenant un moyen de restriction de longueur d'onde (35) limitant une bande de longueur d'onde de la lumière d'éclairage pour éclairer dans la région ultraviolet ou la région visible,
un moyen de transmission d'éclairage (11) transmettant la lumière d'éclairage vers une cible d'observation (5),
une section d'insertion d'endoscope (10a) comprenant un moyen de capture d'images (12) capturant une image d'une lumière fluorescente ou de réfection émise depuis la cible d'observation (5), et
un moyen de génération d'images (51) générant une image de synthèse d'une image fluorescente par la fluorescence et d'une image normale par la lumière de réflexion en se basant sur le signal de capture d'images obtenu par le moyen de capture d'images (12), comprenant:
un premier moyen d'écran de protection de lumière (33), qui est formé d'un matériau qui ne possède pas de propriété de transmission pour la lumière ultraviolette et la lumière visible de la bande de longueur d'onde de la lumière d'éclairage, sur un trajet optique entre le moyen d'émission de lumière (31) et le moyen de restriction de longueur d'onde (35);
**caractérisé en ce qu'**il comprend en outre un deuxième moyen d'écran de protection de lumière (34), qui possède une propriété de transmission seulement pour la lumière ultraviolette, sur un trajet optique entre le moyen de restriction de longueur d'onde (35) et le premier moyen d'écran de protection de lumière (33).

2. Appareil endoscopique selon la revendication 1,
dans lequel le moyen de restriction de longueur d'onde (35) est un filtre rotatif (35) dans lequel trois filtres transmettant respectivement la lumière RVB, et un filtre transmettant la lumière ultraviolette sont disposés de manière concentrique.

3. Appareil endoscopique selon la revendication 2,
dans lequel le premier moyen d'écran de protection de lumière (33) est formé d'un premier mécanisme de diaphragme (33) commandé par un signal de modulation de lumière généré à partir d'un signal de capture d'images, et ajuste les quantités de lumière de la lumière ultraviolette et de la lumière RVB de la bande de longueur d'onde de la lumière d'éclairage émises dans la même séquence de trame depuis le filtre rotatif (35).

4. Appareil endoscopique selon l'une quelconque des revendications 2 et 3,
dans lequel le deuxième moyen d'écran de protection de lumière (34) est formé d'un deuxième mécanisme de diaphragme commandé par un signal de modulation de lumière généré à partir du signal de capture d'images, et ajuste une quantité de lumière d'une lumière RVB de la lumière d'éclairage émise dans une séquence de trame depuis le filtre rotatif (35).

5. Appareil endoscopique selon l'une quelconque des revendications 3 et 4,
dans lequel le signal de modulation de lumière est généré sur la base d'un signal d'informations se rapportant à la luminosité d'une image que possède le signal de capture d'images.

6. Appareil endoscopique selon la revendication 1,
dans lequel le moyen de capture d'images (12) comporte un dispositif de capture d'images (13) convertissant de manière photoélectrique la fluorescence et la lumière de réflexion provenant de la cible d'observation (5), et
un filtre (35) coupant une bande de longueur d'onde de la lumière d'excitation présentant un spectre proche de la région ultraviolet de la lumière de réflexion lorsque la cible d'observation (5) est irradiée par la lumière d'éclairage.

7. Appareil endoscopique selon la revendication 1, dans lequel le moyen de capture d'images (12) comporte un dispositif de capture d'images (13) destiné à saisir des images de la fluorescence comportant un filtre (35) coupant une bande de longueur d'onde de la lumière d'excitation présentant un spectre proche de la région ultraviolet de la lumière de réflexion lorsque la cible d'observation (5) est irradiée par la lumière d'éclairage, et destiné à saisir des images de la lumière de réflexion RVB à partir de la cible d'observation (5).

8. Appareil endoscopique selon la revendication 1, dans lequel
le moyen de restriction de longueur d'onde (35) comporte un filtre (35) transmettant la lumière ultraviolette et un filtre transmettant la lumière blanche, et
le moyen de capture d'images (12) comporte un filtre coloré en forme de mosaïque.

9. Appareil endoscopique selon la revendication 1, comprenant en outre :
un commutateur de mode (16) commutant un mode d'image normale dans lequel l'image normale est générée dans le moyen de génération d'images (51) et affichée, et un mode d'observation simultanée dans lequel l'image fluorescente et l'image normale sont synthétisées dans le moyen de génération d'images (51) et affichées.
